# EUROPEAN PATENT SPECIFICATION

(11) **EP 1 378 209 B1**
(45) Date of publication and mention of the grant of the patent: **19.05.2010**
(21) Application number: 02701716.9
(22) Date of filing: 05.03.2002
(51) Int. Cl.: A61C 17/00, A61M 1/00, A61B 19/00

(54) **AUTOGENOUS BONE COLLECTING DEVICE**
GERÄT ZUM SAMMELN VON AUTOGENEM KNOCHENMATERIAL
DISPOSITIF COLLECTEUR D'OS EN AUTOGREFFE

(30) Priority: 06.03.2001 JP 2001061601
(43) Date of publication of application: 07.01.2004
(73) Proprietor: Takahashi, Atsushi, Tsuruga-shi, Fukui 914-0814 (JP)
(72) Inventor: Takahashi, Atsushi, Tsuruga-shi, Fukui 914-0814 (JP)
(74) Representative: Kewitz, Ansgar
(86) International application number: PCT/JP2002/001991
(87) International publication number: WO 2002/069838

(56) References cited:
- EP-A- 0 931 519
- JP-A- 5 137 736
- JP-A- 2000 166 949
- US-A- 5 505 716
- US-A- 6 083 175
- US-B- 4 921 492
- PATENT ABSTRACTS OF JAPAN vol. 2000, no. 09, 13 October 2000 (2000-10-13) & JP 2000 166949 A (TAKAHASHI ATSUSHI; SHINKO RAITO KOGYOSHO:KK), 20 June 2000 (2000-06-20)

## Description

### BACKGROUND OF THE INVENTION

### Field of the Invention

The invention relates to an autogenous bone collecting device; in particular, to a device for collecting speckles of a bone when an implant socket is drilled during a dental implant operation or selectively collecting a bone from a non-implant site and substituting picked up bone to an implant site.

### Description of the Related Art

In a conventional implant operation, an autogenous bone implantation is often applied when the quantity of the bone matter is insufficient to be implanted into an implant site. When an implanted socket is drilled in a jawbone, speckles cut from the jawbone, and blood, saliva, cooling water from a turbine are filtered and selectively collected by a sucking chip of a dental vacuum device via a filter. The picked up bone itself can be directly implanted by a syringe-shaped speckle collecting device. Alternatively, the picked up bone can be implanted after it is moved to a dappen dish and is mixed with other material. However, in clinical application, in view of easy application, osteoconduction, or infection control, the problem generated from the autogenous bone may increase after the picked up bone is moved to another receptacle and mixed with other material. As stated above, during the normal implantation, the speckles cut from the jawbone, used as bone substitute material, is selectively collected by a sucking chip of the dental vacuum device through the filter when the quantity of the bone is insufficient to be implanted such that autogenous bone implantation is used. The picked up spectle is mixed with other material. Furthermore, when collecting the cut speckle without loss, blood or saliva other than normal saline may also be collected when speckles lost in the oral cavity is suctioned. Thus, the picked up bone may be infected.

Patent JP 200 166949 discloses a bone collectiong unit comprising a sucking duct for sucking a liquid objects including bone powder from the oral cavity and which filters the bone powder.

### SUMMARY OF THE INVENTION

To solve the above problem, the purpose of the invention is to enable the picked up speckles to be mixed with other material without moving to the dappen dish or other receptacle such that the bone implant operation can be simplified. In addition, the container for collecting the bone for implant is prevented from falling such that the picked up bone can be collected without loss. Furthermore, the picked up bone can be taken out easily, and the quantity of the picked up bone and that of the material mixed with the picked up bone can be measured easily. Thus, the picked up speckles can be prevented from loss and from infection by saliva.

To attain the above object, the invention provides a device for collecting bone from an oral cavity. The device includes a suction duct, a main unit body, a filter member, and a waterproof filter holder. The suction duct extracts liquid from the oral cavity, including at least bone, saliva, and blood. The main unit body includes a suctioning side part and a base end side part, and is coaxially connected to the suction duct. The base end side part, through which the liquid excluding the bone passes, is detachable from the suction duct. The suctioning side part, through which the liquid including at least the bone passes, is detachable from the base end side part. The filter member includes a filter portion, a first joint part and a second joint part. The filter member is detachably disposed in the main unit body, and filters the bone from the liquid. The filter portion is container-shaped. The first joint part is fitted to the base end side part, and the second joint part is fitted to the suctioning side part. The waterproof filter holder is adapted to receive the filter member when the filter member is removed from the main unit body. The area of the bottom portion of the filter holder exceeds that of the filter member such that the filter member can be properly positioned. After speckles in the filter member press fitted to the filter holder is mixed with other substance, it is taken out. To improve application ability, osteoconduction, and infection control, when the autogenous bone is mixed with other material, the speckles are selectively collected by the filter assembled on the sucking chip of the dental vacuum device. When the picked up speckles are mixed with other material, including liquid, held in the waterproof filter holder, its application ability can be improved and the quantity of the picked up bone can be prevented from reducing since it needs not to be moved to the dappen dish.

In a preferred embodiment, the device further includes adhesive tape or magic tape attached at the bottom portion of the filter holder. Thus, when the filter holder is fixed to an operation cuff or an operating table via the adhesive tape or magic tape, the bone substitute material, including the speckles, can be taken out without the need to manually hold the filter holder. Since the filter container for collecting bone is prevented from tipping over or falling, the precious collected bone can be prevented from dispersing and that it facilitates the removal of the picked up bone.

In another preferred embodiment, the filter holder is transparent or translucent with a scale on its inner wall and its outer wall such that a volume received in the filter holder can be easily measured. Thus, it becomes possible to easily grasp in proportion the optimum amount of picked up speckles of bone quantity with other mixed material.

In another preferred embodiment, the device further includes a flexible bone collecting dam that includes a first dam joint portion, a second dam joint portion, and a spike. The first dam joint portion is annular, and is made of flexible resin or is made by pressing simulating annealing wire into insert-shaped flexible resin so as to be deformable. Thus, the first dam joint portion can be adapted to a site for operation. The second dam joint portion surrounds the suctioning side part and communicates with the first dam joint portion, and is cylindrical. The spike is disposed on a bottom of the first dam joint portion, and fixes the flexible bone collecting dam to an incision site. Thus, the first dam joint portion is closely adjacent to the bone, and is disposed around a site that the bone is cut. By such sucking, the saliva can be prevented from sucking so that a maximum of collected bone can be collected without losing the picked up bone.

### BRIEF DESCRIPTION OF THE DRAWINGS

The present invention can be more fully understood by reading the subsequent detailed description and examples with references made to the accompanying drawings, wherein:
Fig. 1 is a cross section of an autogenous bone collecting device as disclosed in the invention;
Fig. 2 is an exploded enlarged view of Fig. 1;
Fig. 3 is an enlarged view of a filter member in Fig. 1, wherein a portion, engaged with a base end side part in Fig. 1, of the filter member is shown in an enlarged manner;
Fig. 4 is a bottom view of the filter member;
Fig. 5 is a side view of the filter member held by a filter holder;
Fig. 6 is a schematic view showing an inner part of the filter holder holding the filter member;
Fig. 7 is a schematic view showing a main unit body, with its suctioning side part being connected to a flexible bone collecting dam, disposed on the alveolar bone;
Fig. 8 is a schematic view according to figure 7, wherein the flexible bone collecting dam corresponds to the alveolar bone;
Fig. 9 is a top view of the flexible bone collecting dam;
Fig. 10 is a front view of the flexible bone collecting dam;
Fig. 11 is a side view of the flexible bone collecting dam; and
Fig. 12 is a cross section of the flexible bone collecting dam along a line A-A in Fig. 9.

### DETAILED DESCRIPTION OF THE INVENTION

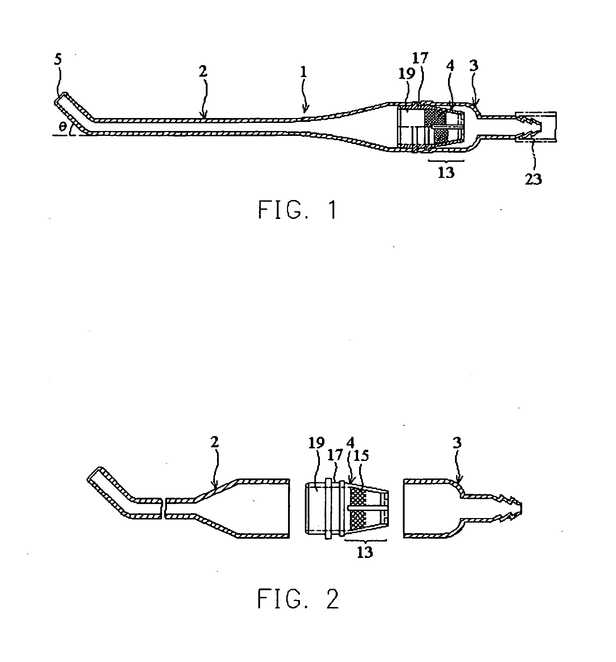

Figs. 1-12 show an embodiment of the invention. In figures, the same reference in different figures represents the same component.

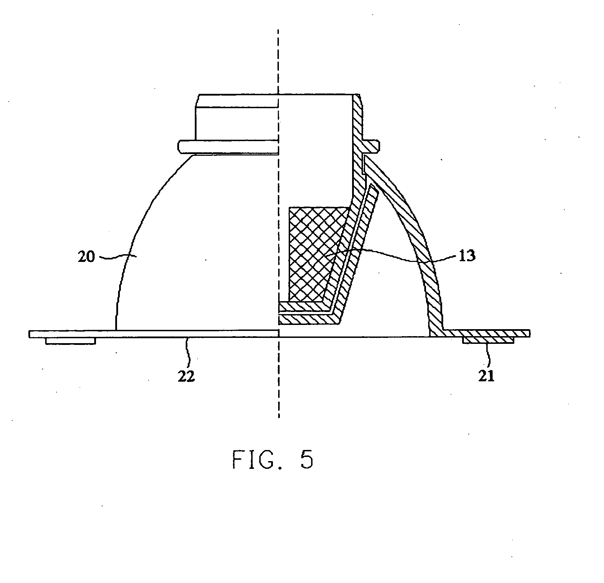

The invention provides a device for collecting bone from an oral cavity. As shown in Fig. 1, the device includes a suction duct 23, a main unit body 1, a filter member 4, and a waterproof filter holder 20 as shown in fig. 5. The suction duct 23 extracts a liquid from the oral cavity, including the bone, saliva, and blood and collects a filtered liquid without the bone. The main unit body 1 includes a suctioning side part 2 and a base end side part 3, and is coaxially connected to the suction duct 23. The base end side part 3, through which the liquid excluding the bone passes, is detachable from the suction duct 23. The suctioning side part 2, through which the liquid including at least the bone passes, is detachable from the base end side part 3.

The filter member 4 includes a filter portion 13, a first joint part 17 and a second joint part 19. The filter member 4 is detachably disposed in the main unit body 1, and filters the bone from the liquid. The filter portion 13 is container-shaped. The first joint part 17 is fitted to the base end side part 3, and the second joint part 19 to the suctioning side part 2.

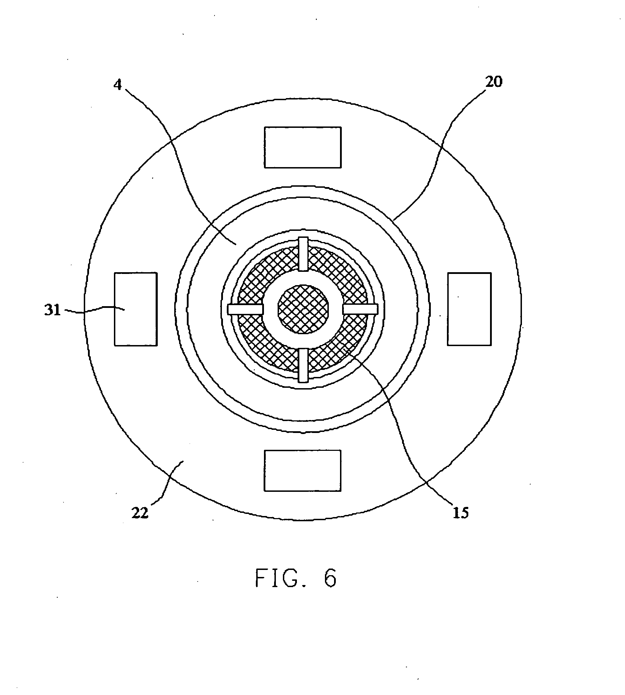

As shown in Fig. 6, the filter member 4 is fitted to the waterproof filter holder 20. The area of the bottom portion of the filter holder 20 exceeds that of the filter member 4 such that the filter member 4 can be properly positioned. Thus, a bone substitute material including at least the bone can be collected from the filter member 4 after the bone is mixed with other material in the filter member 4.

As shown in Figs. 5-6, the device further includes adhesive tape or magic tape 21 attached at the bottom portion of the filter holder 20. Thus, when the filter holder 20 is fixed to an operation cuff or an operating table via the adhesive tape or magic tape 21, the bone substitute material can be collected from the filter member 4 without the need to manually hold the filter holder 20.

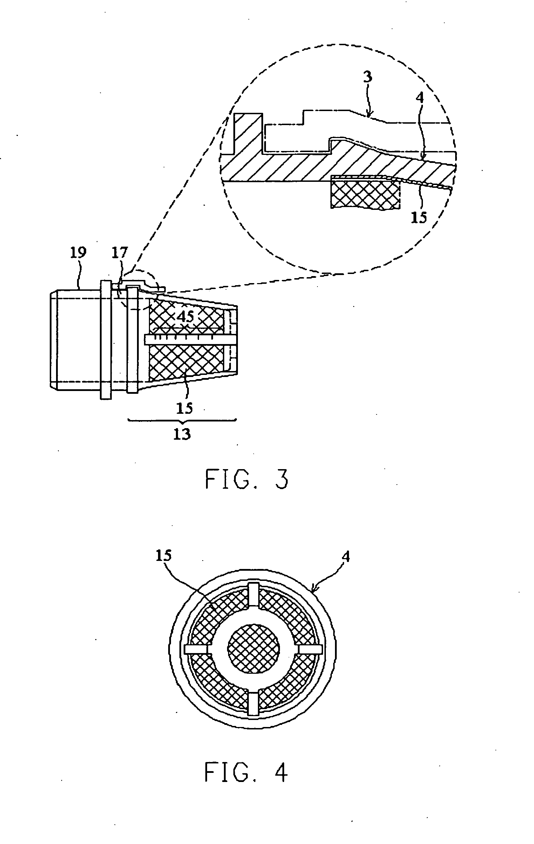

In addition, the filter holder 20 may be transparent or translucent and include a scale 45, as shown in Fig. 3, thereon, such that a volume received in the filter holder 20 can be easily measured.

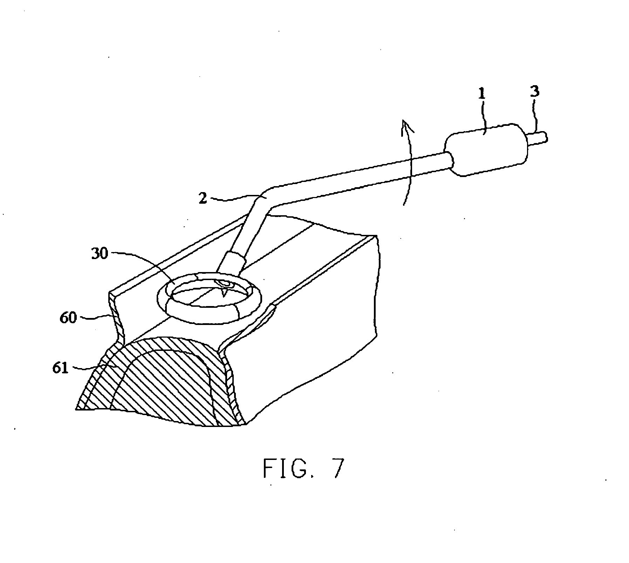

As shown in Figs. 7-12, the device further includes a flexible bone collecting dam 30 that includes a first dam joint portion 31, a second dam joint portion 32, and a spike 33. The first dam joint portion 31 is annular so as to surround a cutting unit 50, and is made of flexible resin so as to be deformable. Thus, the first dam joint portion 31 can be adapted to a site for operation. The second dam joint portion 32 surrounds the suctioning side part 2, communicates with the first dam joint portion 31, and is cylindrical. The spike 33 is disposed on the first dam joint portion 31, and fixes the flexible collecting bone dam 30 to the site for operation (a site for implanting the bone matter). Thus, the first dam joint portion 31 is closely adjacent to the bone such that a maximum of collected bone is collected without loss.

Although the basic structure of the invention is similar to conventional devices, the characteristics of the invention are shown in Figs. 5-8. Specifically, in Figs. 5-6, the filter member 4 for collecting speckles is assembled on the filter holder 20. Also, in Figs. 7-8, the flexible bone collecting dam 30 is disposed at a front end portion 5 of the suctioning side part 2.

The main unit body 1, including the filter member 4, is disposed at a front end of a vacuum device (not shown), and collects speckles to be implanted. After sufficient amount of speckles is obtained, the main unit body 1 can be taken out so as to remove the filter member 4.

The outer surface of the filter member 4, including the filter portion 13, is fitted into the inner surface of the filter holder 20, and is fixed at the filter holder 20 by press fitting an undercut of the first joint portion 17 of the filter member 4. By means of the above operation, since the filter member 4 is formed to fit together with the filter holder 20, the filter member 4 can be properly positioned by the wider base diameter of a bottom portion 22 of the filter holder 20.

Furthermore, since the adhesive tape or magic tape 21 is attached at the bottom portion 22 of the filter holder 20, the filter holder 20 can be easily fixed to the operation cuff or the operating table. Thus, the filter member 4 can be prevented from falling or being inclined, precious bone is collected without loss and can be taken out easily.

In addition, since the inner surface of the filter holder 20 is waterproof, the bone and other mixed material can be held in the filter member 4 without leaking out through a mesh 15. Thus, in a case in which the bone matter is directly mixed with other substitute material for promoting bone construction of the implant site, the bone needs not to be moved to other containers.

Furthermore, since the inner or outer surface of the filter holder 20 is transparent or translucent, the amount received therein can be easily measured by the scale 45. Thus, the amount of the bone to be mixed with other material can be optimized. That is, it becomes possible to easily grasp in proportion the optimum amount of picked up speckles of bone quantity with other mixed material.

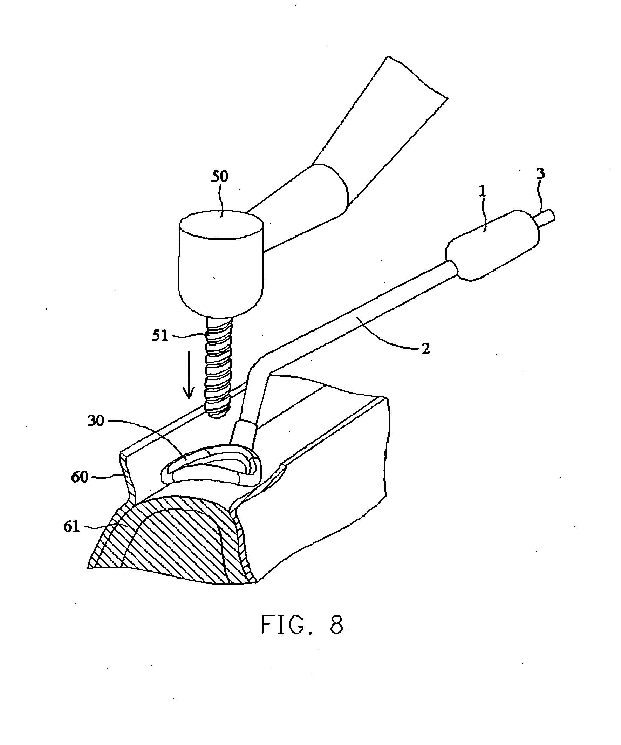

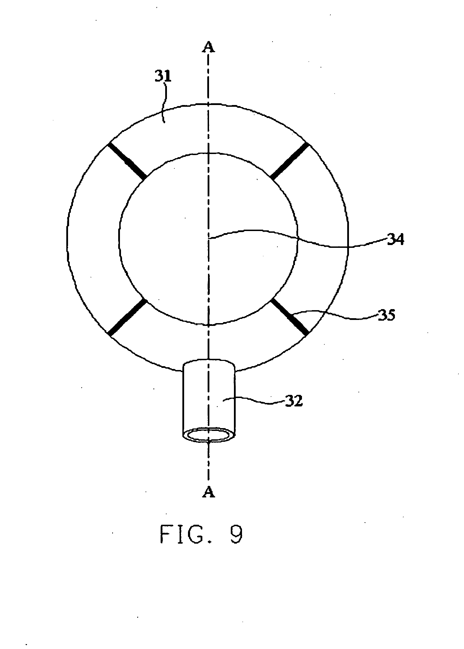

Next, as shown in Figs. 7-8, when collecting the autogenous bone from the oral cavity, the flexible bone collecting dam 30 shown in Figs. 9-11 is assembled at the front end portion 5 via the second dam joint portion 32. The first dam joint portion 31 is positioned around a site to be excavated. The flexible bone collecting dam 30 can be fixed securely by the spike 33. The main unit body 1 is lifted upwardly in the direction shown by the arrow in Fig. 8. Since the bottom portion of the first dam joint portion 31 corresponds to alveolar corrical bone 61, the bone can be prevented from loss by a space 34. At this time, mucoperiosteal flap 60 can be removed by the first jam joint portion 31 so as to be clearly seen. Thus, soft tissue can be prevented from damage by a cutting drill 51.

A mark 35 on the flexible bone collecting dam 30 can correctly indicate the position of the bone to be cut.

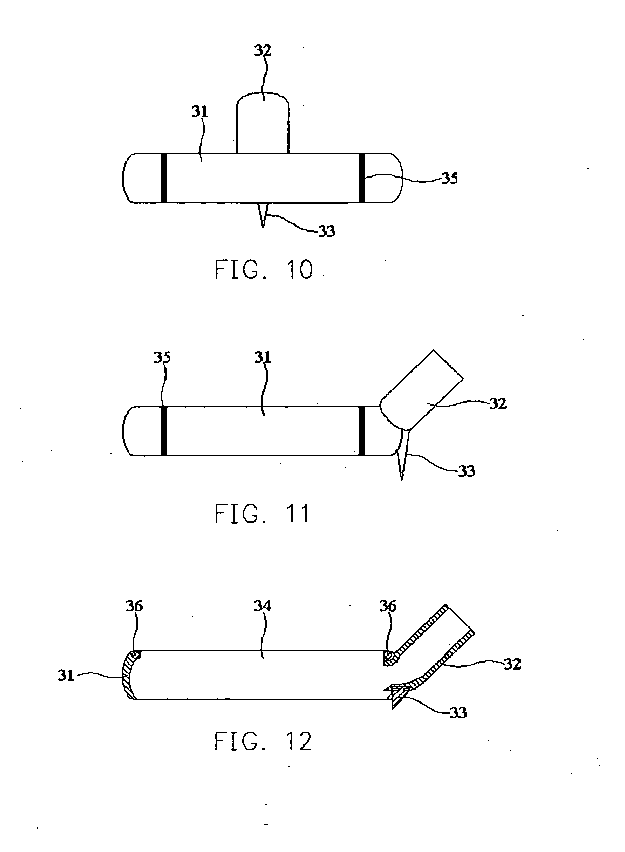

Furthermore, as shown in Fig. 12, a metallic annealing wire 36 can be previously inserted into the first dam joint portion 31. Thus, the bottom portion of the first dam joint portion 31 can easily conform to any shape by pressing so as to correspond to the complex shape of the alveolar corrical bone 61.

Furthermore, the material and assembly manner of the second dam joint portion 32 are not limited, nor are the material and shape of the metallic annealing wire 36.

## Claims

1. A device for collecting bone comprising:
a suction duct (23) for extracting a liquid comprising at least the bone from an oral cavity and collecting a filtered liquid without the bone;
a main unit body (1), including a suctioning snide, part (2) and a base end side part (3), the base end side part (3) being coaxially connected to the suction duct, wherein the base end side part, through which the liquid excluding the bone passes, is detachable from the suction duct, and the suctioning side part, through which the liquid comprising at least the bone passes, is detachable from the base end side part;
a filter member (4), including a filter portion (13), a first joint part (17) and a second joint part (10), detachably disposed in the main unit body for filtering the bone from the liquid, wherein the filter portion is container-shaped, and the first joint part is fitted to the base end side part, and the second joint part is fitted to the suctioning side part,
a waterproof filter holder (20) adapted to stand on an operation cuff or an operating table and wherein the filter member is adapted to be fitted to the waterproof filter holder (20) when the filter member is removed from the base end side part, wherein the waterproof filter holder has a waterproof inner surface adapted to receive an outer surface of the filter member, wherein the area of the bottom portion of the filter holder exceeds that of the filter member to position the filter member properly..

2. The device as claimed in claim 1, further comprising adhesive tape or magic tape (21) attached at the bottom portion of the filter holder, such that the bone substitute material can be collected from the filter member when the filter holder is fixed to an operation cuff or an operating table thereby.

3. The device as claimed in claim 1, wherein the filter holder is transparent or translucent, and the filter holder includes a scale thereon such that a volume received in the filter holder can be easily measured.

4. The device as claimed in claim 1, further comprising a flexible bone collecting dam (30) connected to the suction side part of the collecting bones, comprising:
a first annular dam joint portion (31) made of flexible resin so as to be deformable, whereby the first dam joint portion can be adapted to a site for operation and
a second dam joint portion (32), surrounding the suctioning side part and communicating with the first dam joint portion, being cylindrical.
5. The device as claimed in claim 4, wherein the flexible bone collecting dam further comprises a spike (33), disposed on a bottom portion of the first dam joint portion, for fixing the flexible bone collecting dam to the site for operation, wherein the first dam joint portion is closely adjacent thereto.

## Patentansprüche

1. Ein Gerät zum Einsammeln von Knochen umfassend:
Einen Ansaugkanal (23) zum Extrahieren einer Flüssigkeit umfassend zumindest den Knochen von einer Mundhöhle und zum Einsammeln einer gefilterten Flüssigkeit ohne die Knochen;
Eine Hauptkörpereinheit (1) umfassend ein saugendendes Seitenteil (2) und ein Basisendenseitenteil (3), das Basisendenseitenteil (3) ist koaxial mit dem Ansaugkanal verbunden, wobei das Basisendenseitenteil, durch welches die Flüssigkeit ohne die Knochen passiert lösbar vom Ansaugkanal ist, und das Saugseitenteil, durch welches die Flüssigkeit umfassend zumindest die Knochen fließt, ist lösbar von dem Basisendenseitenteil;
ein Filterelement (4) umfassend einen Filterbereich (13), ein erstes Verbindungsstück (17) und ein zweites Verbindungsstück (10), die lösbar in der Hauptkörpereinheit angeordnet sind, um den Knochen von der Flüssigkeit zu filtern, wobei der Filterbereich eine Behälterform aufweist und das erste Verbindungsstück in das Basisendenseitenteil eingepasst und das zweite Verbindungsstück in das Saugseitenteil eingepasst ist;
ein wasserdichten Filterhalter (20) angepasst, um auf einen Operationsschlag oder einem Operationstisch zu stehen, und wobei das Filterelement ausgebildet ist, um in den wasserdichten Filterhalter (20) eingeführt zu werden, wenn das Filterelement von Basisendenseitenteil entfernt wurde, wobei der wasserdichte Filterhalter eine wasserdichte innere Oberfläche aufweist, die ausgebildet ist, um eine äußere Oberfläche des Filterelementes aufzunehmen, wobei der Bereich des Bodenteils des Filterhalters den des Filterelements überschreitet, um das Filterelement richtig zu positionieren.

2. Das Gerät nach Anspruch 1 weiterhin umfassend ein adhäsives Band oder ein magnetisches Band (21), das am Bodenbereich des Filterhalters befestigt ist, so dass das knochenersetzende Material vom Filterelement gesammelt werden kann, wenn der Filterhalter an einem Operationsstulpe oder einem Operationstisch befestigt ist.

3. Das Gerät nach Anspruch 1 wobei der Filterhalter transparent oder transluzent ist und der Filterhalter enthält eine Skala, wodurch ein Volumen, das durch den Filterhalter empfangen wurde, einfach gemessen werden kann.

4. Das Gerät nach Anspruch 1 weiterhin umfassend einen flexiblen Knochensammeldamm (30), der an dem Saugseitenteil des Knochensammlers befestigt ist; umfassend:
Einen ersten ringförmigen zusammengefügten Dammbereich (31) der aus flexiblem Harz gefertigt ist, um somit deformierbar zu sein, wobei der erste zusammengefügte Damm für eine Stelle für die Operation angepasst werden kann und
einen zweiten zusammengefügten Anschlussbereich (32), der das Ansaugseitenteil umgibt und mit dem ersten Dammanschlussbereich zylindrisch verbunden ist.

5. Das Gerät nach Anspruch 4 wobei der flexible Knochensammeldamm weiterhin einen Stachel (33), der am Bodenbereich des ersten Verbindungsdammbereiches angeordnet ist, umfasst, um den flexiblen Knochensammeldamm auf vor Ort für die Operation zu befestigen wobei der erste Verbindungsdammbereich eng daran angrenzend ist.

## Revendications

1. Un dispositif de collecte d'os comportant:
un conduit d'aspiration (23) pour l'extraction depuis une cavité orale d'un liquide comportant au moins l'os et collectant un liquide filtré dépourvu d'os ;
un corps principal (1), comportant une partie latérale de succion (2) et une partie latérale terminale (3), la partie latérale terminale (3) étant axialement fixée au conduit d'aspiration, dans lequel la partie latérale terminale, par lequel circule le liquide dépourvu d'os, est détachable du conduit d'aspiration, et la partie latérale de succion, par laquelle circulant le liquide comportant l'os, est détachable de la partie latérale terminale;
un corps de filtrage (4), comportant un élément de filtrage (13), une première élément de raccord (17) et un second élément de raccord (10), disposés de manière amovible dans le corps principal pour le filtrage de l'os au sein du liquide, dans lequel l'élément de filtrage présente une forme de container, le premier élément de raccord est disposé sur la partie latérale terminale et le second élément de raccord est disposé sur la partie latérale de succion ;
un support de filtre imperméable (20) adapté pour être disposé sur un manchon d'intervention ou une table d'intervention et dans lequel le corps de filtrage est adapté au support de filtre imperméable (20) lorsque le corps de filtrage est détaché de la partie latérale terminale, dans lequel le support de filtre imperméable présente une surface interne adapté à la réception d'une surface exterieure du corps de filtrage, dans lequel la surface de la partie inférieure du support de filtrage est plus grande que celle du corps de filtrage dans le but d'un positionnement convenable du corps de filtre.

2. Le dispositif selon la revendication 1, comportant en outre un ruban adhésif ou magique (21) fixé à la partie inférieure du support de filtrage, de telle manière que le matériau de remplacement de l'os peut être collecté dans le corps de filtrage lorsque le support de filtre est fixé à une manchette d'opération ou sur une table d'opération.

3. Le dispositif tel que revendiqué dans la revendication 1, dans lequel le support de filtre est transparent ou translucide, et dans lequel le support de filtre comporte une échelle disposée pour permettre une mesure aisée du volume collectée dans le support de filtre.

4. Le dispositif selon la revendication 1, comportant en outre un élément flexible de retenue d'os (30) fixée à la partie latérale de succion de l'os aspiré,:
une première partie annulaire de raccord avec l'élément de retenue (31) réalisée au moyen d'une résine flexible pour permettre une déformation, par laquelle la première partie de raccord avec l'élément de retenue peut être adapté à un site operatoire et
une seconde partie cylindrique de raccord avec l'élément de retenue (32) entourant la partie latérale de succion et communiquant avec la première partie de raccord avec l'élément de retenue.

5. Le dispositif selon la revendication 4, dans lequel l'élément flexible de retenue d'os comporte en outre une pointe (33), disposée sur une partie inférieure de la première partie de raccord avec l'élément de retenue, pour fixer l'élément flexible de retenue d'os sur le site de l'intervention, de manière proche à la première partie de raccord avec l'élément de retenue.
